Europäisches Patentamt

European Patent Office

Office européen des brevets

(11) Publication number: **0 160 801 B1**

# EUROPEAN PATENT SPECIFICATION

(45) Date of publication of patent specification: **25.09.91**

(51) Int. Cl.⁵: **A61N 1/368**

(21) Application number: **85102404.2**

(60) Publication number of the earlier application in accordance with Art.76 EPC: **0 058 603**

(22) Date of filing: **09.02.82**

(54) Implantable medical device power source depletion indicators.

(30) Priority: **17.02.81 US 235069**

(43) Date of publication of application:
**13.11.85 Bulletin 85/46**

(45) Publication of the grant of the patent:
**25.09.91 Bulletin 91/39**

(84) Designated Contracting States:
**DE FR IT NL SE**

(56) References cited:
**US-A- 3 661 158**

(73) Proprietor: **MEDTRONIC, INC.**
**3055 Old Highway Eight**
**Minneapolis Minnesota 55440(US)**

(72) Inventor: **Herpers, Lodewijk-Jozef**
**Graverstraat 153**
**6466 KV Kerkrade(NL)**

(74) Representative: **Polus, Camille et al**
**c/o Cabinet Lavoix 2, Place d'Estienne d'Orves**
**F-75441 Paris Cedex 09(FR)**

## Description

### BACKGROUND OF THE INVENTION

#### Field of the Invention

This invention relates to operating mode and parameter changes in artifical implantable medical device, e. g. cardiac pacemakers, in response to and to indicate depletion of the device's sensing power source.

#### Description of the Prior Art

The implantable cardiac pacemaker, shown in U.S. Patent No. 3,057,356 and subsequent patents permits innocuous, painless, long-term cardiac stimulation at low power levels by utilizing a small completely implanted transistorized and battery operated pulse generator connected via a flexible lead bearing an electrode directly in contact with cardiac tissue. Most pulse generators consist of a stimulating circuit and a sensing circuit both of which draw current from the battery. In the presence of complete heart block, an asynchronous pulse generator with only a stimulating circuit may be used, however, in most instances, noncompetitive triggered or inhibited pulse generators having the sensing circuit are preferred and dominate the pacemaker market. The demand, synchronous or triggered pulse generators are especially useful in patients with spontaneous cardiac activity because of their ability to sense intrinsic cardiac rhythm (atrial or ventricular depending on variety and electrode position), and to alter the pacemaker output accordingly. Such pacemakers are shown for example, in U.S. Patent Nos. 3,253,596 (P-wave synchronous), 3,478,746 (ventricular inhibited) and are described in the pacing literature.

More recently, attention has been paid to the physiological aspects of cardiac pacing therapy and particularly to pacing systems for maintaining synchronous atrial and ventricular depolarization of the heart. In early atrial synchronized (or A-V synchronous) pacing, atrial depolarization is sensed through one electrode, and after an appropriate delay the ventricle is paced through a different electrode, thereby restoring the normal sequence of atrial and ventricular contraction and allowing the pacer to respond to physiologic needs by increasing its rate. Below a predetermined minimal atrial rate, however, the pacemaker reverts to its basic ventricular pacing rate. In atrial synchronous, ventricular inhibited pacers of the type described in U.S. Patent Nos. 4,059,116 and 3,648,707, the ventricular depolarizations are also sensed and inhibit or reset the timing of the ventricular stimulating pulse generator.

A more complex method of restoring synchrony is by the atrial ventricular sequential pacing of the type described in U.S. Patent No. 3,595,242 and subsequent patents which possess atrial and ventricular pulse generators and associated electrodes and a ventricular sensing circuit. In atrial ventricular sequential pacing, the atria and ventricles are paced in proper sequence, the atrial and ventricular pulse generator timing circuits being reset on sensing spontaneous ventricular activity.

Finally, U.S. Patent N° 4,312,355 by Herman D. Funke, (assigned to a subsidiary company of the assignee of the present invention), discloses a pacemaker which, if required, may stimulate the atrium and/or ventricle on demand and which is able to maintain synchrony as the sensed atrial rate increases. A pacemaker of this type is capable of distinguishing between bradycardia and normal heart function and to provide atrial and/or ventricular pacing in the following modes: inhibited in the case where the atrium and ventricle beat at a sufficient rate; atrial demand in instances where the atrium is beating at an insufficient rate and must be stimulated whereas the ventricle properly follows; atrial synchronous when the atrium depolarizes at a sufficient rate but the ventricle does not follow within a prescribed A-V interval; and demand in both chambers when neither the atrium and the ventricle spontaneously depolarize at the desired rate.

All of the demand pacemakers of the types described above comprise ventricular and/or atrial timing circuits which may be a simple oscillator of the early designs or the complex, programmable, digital timing circuit of the type disclosed, for example, in the commonly assigned U.S. Patent No. 4,230,120, an analog sense amplifier circuit of the type disclosed, for example, in U.S. Patent N° 4,265,551 by Marc T. Stein, and an analog output circuit of the type disclosed, for example, in the copending U.S. Serial No. 997,826 filed November 6, 1978 in the name of David L. Thompson, all assigned to the assignee of the present invention. The inputs of the respective sense amplifiers and the output capacitances of the output circuits are commonly coupled to the respective atrial or ventricular sense amplifiers and through pacing leads to the electrodes coupled to the patient's heart.

Such medical devices as the demand pacemakers of the types described above are powered by depletable power sources or batteries which deplete from a beginning of life (BOL) to an end of life (EOL) voltage and current condition. The depletion reduces the current and voltage available to power the various components of the pulse generator and provide an adequate stimulation energy leading to either loss of capability of the device or capture of the patient's heart. To alert the patient or physician of the condition of the battery, prior art pulse generators have provide end-of-life indicator circuits, usually activated by a magnetically actuated reed switch, which cause the pulse generator to operate in an asynchronous mode at a rate which differs from the rate provided at BOL in proportion to the power source depletion or to a predetermined EOL rate upon depletion or to a predetermined EOL rate upon depletion to a selected EOL energy level. Such prior art pulse generators of the type described in the aforementionned U.S. Patent N° 4,230,120 possess such EOL circuitry for indicating to the physician the state of depletion of the power source. In a multi-programmable multi-mode medical device of the type described herein, several modes and operating parameters of the device may be selectively programmed. In the atrial and ventricular pacemaker of the type described herein, the energy drain on the power source is accentuated by the complexity and number of components in the circuitry and by the A-V sequential pacing output pulses which over the same period of time, at full pacing, draw current more rapidly from the battery than a conventional single chamber demand pacemaker. These characteristics of the pulse generator in which the present invention is embodied require a more complex and graduated manner in which the power source depletion may be monitored under several operating conditions.

It is known from US-A-3,661,158 a medical device including an atrial output stage for providing atrial stimulating pulses at a recurring rate interval, said output stage including an atrial capacitor, means for detecting ventricular depolarizatin and means for providing and applying a control signal to the means for detecting vertricular depolarization to disable this detecting means for a predetermined period of time starting in timed relation to the generator of an atrial pace initiate pulse, so that the means for detecting ventricular depolarization, cannot sense said atrial simulation pulse and mistake it for a ventricular depolarization.

## SUMMARY OF THE INVENTION

Accordingly, in recognition of the above-stated characteristics of the more complex medical devices, the present invention provides a battery depletion indicator which can be selectively interrogated to cause the medical device to operate in pre-selected modes and rates which indicate the mode in which the device is programmed and the depletion level of the power source.

According to the invention a programmable medical device is provided including :

(a) an atrial output stage for providing atrial stimulating pulses at a recurring rate interval, said output stage including an atrial capacitor;

(b) means for detecting ventricular depolarizations;

(c) means for applying external programming signals to the programmable device for programming variable operational parameters and variable modes;

(d) programmable digital control means including :

(i) means for storing variable digital data derived from said externally applied programming signals corresponding to a lower rate at which the pulse generator will pace if neither P (atrial) waves nor R (ventricular) waves are sensed within the escape intervals established by said lower rate data and for storing the desired programmable interval between the production of an atrial and a subsequent ventricular stimulation pulse (A-V interval);

(ii) means for providing an atrial pace initiate signal to said atrial output stage to initiate an atrial stimulating pulse if no naturally occuring P wave is sensed within the escape interval established by the lower rate data, the atrial stimulating pulse being the discharge of said atrial capacitor;

(iii) means for providing and applying a control signal to the means for detecting ventricular depolarizations to disable this detecting means for a predetermined period of time starting in timed relation to the generation of an atrial pace initiate pulse, so that the means for detecting ventricular depolarizations cannot sense said atrial stimulation pulse and mistake it for a ventricular depolarization, and

(iv) means for generating a fast recharge control signal, to control the rechargte cycle.

The foregoing and additional advantages and characterizing features of the present invention will become clearly apparent upon reading of the ensuing detailed description of an illustrative embodiment thereof together with the included drawings depicting this theme.

## BRIEF DESCRIPTION OF THE DRAWINGS

FIG. 1 is a block circuit diagram of the dual chamber pacemaker of the present invention;

FIG. 2 is a block circuit diagram of the digital controller circuit employed in the FIG. 1 circuit; and

## DESCRIPTION OF THE PREFERRED EMBODIMENT

The depletion mode operation of the pulse generator of the present invention will be explained in detail after the following descriptions of the dual chamber DDD pacemaker in which it is embodied.

The dual chamber pacemaker of the present invention possesses five basic components in addition to the necessary power supply and leads for conducting electrical signals between the patient's heart and the pacemaker pulse generator. These components are an atrial sense amplifier, a ventricular sense amplifier, an atrial output stage, a ventricular output stage, and a digital controller circuit possessing programmable memory and logic circuits which time the production of atrial and/or ventricular stimulating output pulses as a function of the P waves sensed by the atrial sense amplifier, R waves sensed by the ventricular sense amplifier, parameter and mode data stored within the memory, the status of the power source voltage, and the condition of a reed switch which may be effected by an external magnetic field. It is contemplated that in the preferred embodiment of this invention, the pulse generator would be contained within a sealed metallic container electrically connected to the output stages to act as an indifferent electrode and a pair of output connectors or terminals adapted to be coupled by way of leads from the output terminals extending to the atrium and ventricle of the patient's heart.

It is contemplated that the dual chamber pacemaker of the present invention may automatically operate in several different pacing modes depending on the presence or absence of sensed atrial and/or ventricular depolarizations, that is P waves and/or R waves, in a manner described generally for example in the abovementioned U.S. Patent N° 4,312,355.
In this context, it is contemplated that several modes of operation may automatically take place depending on the condition of the patient's heart.

Generally, if neither P-waves nor R-waves are present in the atrial and ventricular escape intervals set by the timing circuitry of the pacemaker, it will function in the dual chamber, A-V sequential fixed rate mode (DOO mode as this mode and others referred to herein are designated in the Report of the Inter-Society Commission for heart Disease Resources in Circulation, Vol. L., October, 1974.) If P-waves are not present, but R-waves recur from time to time within the A-V delay interval, the device operates in the A-V sequential demand or DVI mode. If P-waves are present, this device operates in the atrial synchronous or VAT mode in the absence of R-waves sensed within the A-V escape interval or in the atrial synchronous ventricular inhibited (ASVIP) or VDT/I mode when R-waves recur within the A-V interval.

In addition it is contemplated that the dual chamber pacemaker of the present invention may be externally programmed to operate in a number of different modes including: the fully automatic dual chamber, or DDD mode described above, in which the atrial and ventricular sense amplifiers and the atrial and ventricular output stages are fully operational; in the atrial-ventricular sequential pacing mode, or DVI mode, wherein atrial and ventricular stimulating pulses are provided in timed relationship to one another in the absence of sensed ventricular depolarization; in the atrial synchronous, ventricular inhibited (ASVIP) mode, or the VDT/I mode, wherein the sensed atrial depolarizations cause the pulse generator to deliver a ventricular stimulation pulse unless a spontaneous or conducted ventricular depolarization is detected by the ventricular sense amplifier prior to the timing out of a suitable A-V delay period; or the ventricular demand pacing mode, the VVI mode, wherein the atrial sense amplifier and atrial output stages are not employed.

In addition it is contemplated that the pulse generator can be programmed to operate in the atrial asynchronous mode (AOO), the ventricular asynchronous mode (VOO), or the atrial ventricular asynchronous mode (DOO), by selectively programming pulse width to zero and sensitivity to infinite to arrive at these resulting combinations.

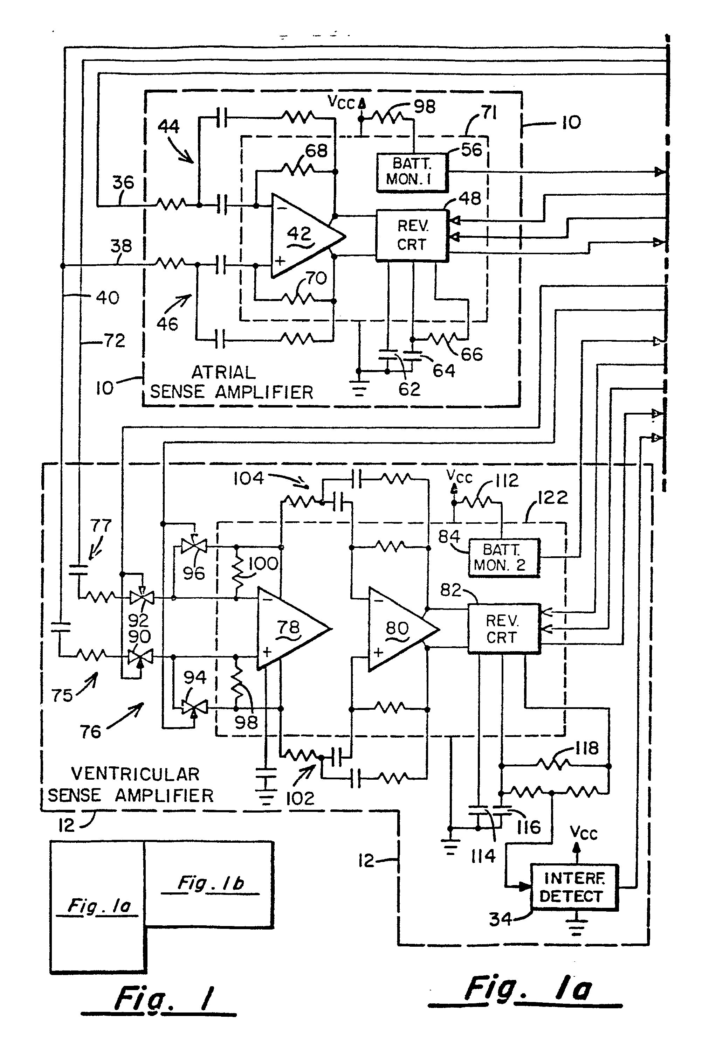

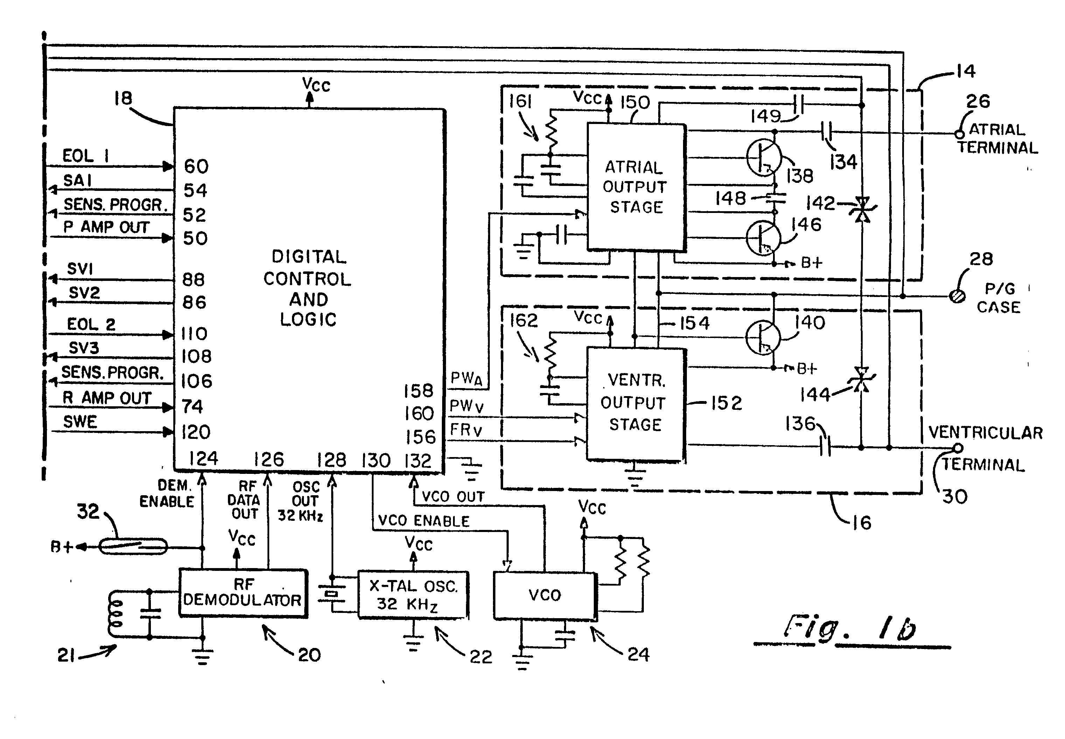

Turning now to FIG. 1, there is shown an atrial sense amplifier 10, ventricular sense amplifier 12, atrial output stage 14, ventricular output stage 16, digital control and logic circuit 18, and three further circuits, the RF demodulator circuit 20 for receiving remotely applied programming signals and magnetic field test signals, a crystal oscillator 22 for providing the basic clock frequency for the digital control and logic circuit 18, and a voltage controlled oscillator (VCO) circuit 24 for timing certain operations of the digital control and logic circuit 18. The atrial sense amplifier 10 is coupled between the atrial lead terminal 26 and the case terminal 28 for sensing atrial depolarizations, or P waves. The ventricular sense amplifier 12 is similarly coupled to the case terminal 28 and the ventricular pacing lead terminal 30, for sensing ventricular depolarizations, or R waves. The digital control and logic circuit 18 is coupled to the output terminals of the atrial and ventricular sense amplifiers to receive the atrial amplifier output signal at terminal 50 and the ventricular amplifier output signal at terminal 74, to process the signals in accordance with the mode to which the pacemaker is programmed and the parameters of atrial and ventricular timing escape intervals and to produce, if appropriate, atrial pace initiate signals PWA and ventricular pace initiate signals PWV-which are respectively coupled to input terminals of the atrial output stage 14 and the ventricular output stage 16 to initiate respective atrial and ventricular stimulating pulses. The production of the atrial and/or ventricular initiate pulses is dependent upon the presence or absence of sensed atrial and/or ventricular depolarizations of the heart within certain escape intervals established by the parameter data stored in memory within the digital control and logic circuit 18 and dependent upon whether or not the reed switch 32 is open or closed by the application of an external magnet or on the condition of interference detector 34 within the ventricular sense amplifier 12 which responds to noise signals picked up by the ventricular sense amplifier. Ignoring for the moment the possibility of the closure of the reed switch 32 or interference, and turning to the operation of the circuit in the DDD mode, the pulse generator will pace at a programmable lower rate if neither P waves nor R waves are sensed within the escape intervals established by lower rate data stored in memory. The A-V interval between the production of atrial and ventricular initiate signals by the digital control and logic circuit 18 is similarly programmable and, under these conditions, the total operation of the pacemaker is characterized as A-V sequential, demand pacing.

If a P wave is sensed by the atrial sense amplifier 10 within the programmed atrial escape interval, (corresponding to a programmble lower rate), the digital control and logic circuit 18 will not produce an atrial pace initiate signal, but instead will commence the timing of the A-V interval. If an R wave is sensed by the ventricular sense amplifier 12 prior to the expiration of the A-V interval, the ventricular pacing initiate signal is similarly not produced, and all timing intervals will be reset. But if an R wave is not sensed prior to the completion of the A-V interval, a ventricular pace initiate pulse will be provided by the digital control and logic circuit to initiate the production of a ventricular pacing stimulus at the end of the delay.

If the memory within the digital control and logic circuit 18 is programmed to the VVI mode, the atrial sense amplifier output signal is ignored, and the atrial output initiate signal is not produced. Thus, the digital control and logic circuit 18 responds only to R waves sensed by the ventricular amplifier 12 and produces only ventricular pace initiate signals in the absence of an R wave occuring prior to the expiration of the ventricular escape interval.

If the device is programmed in the DVI mode, the atrial output signal of the atrial sense amplifier 10 is similarly ignored. However, the atrial output stage and the ventricular output stage receive atrial and ventricular pace initiate signals at the programmable lower rate and separated by the A-V interval. If an R wave is sensed during the escape interval and following the refractory period of the ventricular sense amplifier, the lower rate timing is reset. If an R wave is sensed prior to the completion of the A-V interval and following the delivery of an atrial stimulating pulse to the heart, then the ventricular pace initiate signal is inhibited or not delivered, and the lower rate escape interval is again reset.

In the atrial synchronous ventricular inhibited, or VDT/I mode, P waves recurring at a rate exceeding the programmable lower rate, are sensed by the atrial sense amplifier and processed by the digital control and logic circuit 18 to commence the A-V timing interval. Again, if an R wave is sensed prior to the completion of the A-V interval, the ventricular pacing output is inhibited and all timing circuits will be reset. But, it an R wave is not sensed prior to the completion of the A-V interval, a ventricular pacing stimulus will be provided in response to a ventricular pace initiate signal at the end of the A-V interval. A sensed R wave occurring within the programmed lower rate escape interval and after the refractory period since the last ventricular depolarization or stimulating pulse, will be processed by the digital control and logic circuit 18 to restart the lower rate timing interval.

The above modes of operation may be selected by the physician to conform the operation of the pacemaker to the patient's condition or the condition of the atrial or ventricular pacing leads. Ordinarily it would be expected that the pacemaker would be left operating in the fully automatic dual chamber or DDD mode, with the lower rate interval, A-V timing interval and other parameters of operation of the device being selected and programmed to conform to the patient's condition.

Turning now to the specific elements shown in FIG. 1, the atrial sense amplifier 10 is coupled to the atrial lead 26 and the pulse generator 28 through conductors 36 and 38, 40 respectively. The conductors 36 and 38 are coupled to the negative and positive input terminals of differential amplifier 42 to a balanced resistance and capacitive network circuit 44 and 46 connected between the input and output terminals of the differential amplifier 42. The differential output signal of the amplifier 42 is coupled to a reversion circuit 48 which is designed to detect natural heart depolarizations in the presence of continuous noise signals. The reversion circuit 48 responds to the amplified signal to produce a P wave output signal which is applied to the input terminal 50 of the digital control and logic circuit 18.

Atrial sense amplifier 10 depicted in FIG. 1 also shows a pair of reversion capacitors 62, 64 and a trim resistor 66 coupled to the reversion circuit 48. The differential amplifier 42, the reversion circuit 48, the battery monitor 56 and gain control resistors 68 and 70 can be packaged within a linear circuit chip 71 coupled to source voltage Vcc and system ground. The atrial sense amplifier as described conforms substantially to that depicted and described in the aforementioned U:S. patent N° 4,266,551.

Turning to the ventricular sense amplifier 12, it is coupled by conductors 40 and 72 to the pulse generator case 28 and the ventricular lead 30, respectively, and at its output terminal 74 to the digital control and logic circuit 18. The ventricular sense amplifier contains a FET switch array 76, a first differential amplifier 78, a second differential amplifier 80, a reversion circuit 82, a second battery monitor circuit 84, and various biasing and filtering components. In general terms, the ventricular sense amplifier 12 is designed to detect ventricular depolarizations and to avoid detecting atrial stimulating pulses delivered by the atrial output stage 14. To that end, the ventricular sense amplifier 12 is provided with a FET switch array 76 and the first differential amplifier 78 coupled between the output terminals 28 and 30 and the input terminals of a second differential amplifier 80.

In general terms, the FET switch array 76 possesses a first pair of series switches of transmission gates controlled by a first switch signal SV1 at terminal 88 and a second switch signal SV2 at terminal 86. The transmission gates 90 and 92 are coupled in series with the positive and negative input terminals of differential amplifier 78 and the transmission gates 94 and 96 are coupled in parallel with gain control resistors 98 and 100 coupled between the input and output terminals of amplifier 78. Ordinarily, in the absence of signals SV1 and SV2, the gates 90 and 92 are closed and the gates 94 and 96 are open. Thus, the differential amplifier 78 is ordinarily coupled to series connected RC filter circuits 79, 77 and the conductor 72 and 40 to the ventricular lead 30 and pulse generator case 28 respectively, and the gain of the differential amplifier 78 is controlled by the value of the resistors 98 and 100. Thus, signals appearing on the conductors 40 and 72 are differentially amplified in the balance differential amplifier 78 and coupled to RC circuits 102 and 104 to the second differential amplifier 80. As in the atrial sense amplifier 10, the output signals are coupled to a reversion circuit 82 to provide an R wave amplifier output signal at the terminal 74 of the digital control and logic circuit 18.

The signals SV1 and SV2 are generated just prior (e.g. 2 clock pulses earlier) to the generation of an atrial pace initiate pulse PWA which is delivered to the atrial output stage 14. The signals SV1 and SV2 are applied to the transmission gates 90-96 to open the gates 90 and 92 and close the gates 94 and 96 to disconnect the input terminal of the differential amplifier 78 from the conductors 40 and 72 and lower the amplifier's gain by shorting out the resistors 98 and 100. After a certain interval, such as 8 ms, the signal SV1 ceases, thus restoring the connection of the differential amplifier 78 to the conductors 40 and 72. A short time thereafter, such as 3.9 ms later, the signal SV2 ceases and the full gain of the differential amplifier 78 is restored. This operation insures that the atrial output pulse reflected on the pulse generator case terminal and terminal 30 does not itself cause the ventricular sense amplifier to mistakenly produce an output pulse.

The reversion circuit 82 also includes circuitry which upon receiving an appropriate programming signal from the digital control and logic circuit 18 can adjust the sensitivity of the ventricular sense amplifier 12 through a signal on terminal 106, and circuitry for responding to a further switch signal SV3 at terminal 108 for blanking the ventricular sense amplifier 12 following the sensing of an earlier ventricular depolarization or the production of a ventricular pace initiate signal. The ventricular sense amplifier 12 further comprises a second battery monitoring circuit 84 for producing a second end-of-life signal EOL2 at terminal 110 when source voltage Vcc falls below a further lower reference voltage Vref2 established by the monitoring circuit 84. In addition, the ventricular sense amplifier 12 possesses the first and second reversion circuit capacitors 114 and 116, sensitivity trimming resistors 118 and an interference detecting circuit 34 for producing a signal SWE at terminal 120 when interference causes the capacitor 116 of reversion circuit 82 to become charged to a certain level. The control signal SWE at terminal 120 indicates to the digital control and logic circuit 18 that interference is present and causes it to provide certain control signals to insure that the reversion circuit 82 does not itself falsely produce an R wave output pulse at terminal 74 on the resumption of applied interference signals following the connection of the differential amplifier 78 to terminals 28 and 30 in the manner described above.

It is contemplated that the first and second differential amplifier 78 and 80, the reversion circuit 82, the battery monitor circuit 84 and certain of the resistances would be constructed in a linear IC circuit 122. Filter and bias circuit components 75, 77, 102, 104, 112. 118 are selected to provide the appropriate frequency response and specification as is well known in the art.

The reed switch 32 is shown coupled between the input terminal 124 and battery voltage B + . The closure of the reed switch 32 applies battery voltage to the terminal 124 and additional control and logic circuit 18 responds thereto to perform certain functions to be described in greater detail later, and to the RF demodulator 20 for enabling the RF demodulator 20 to demodulate applied RF signals detected in the tank circuit 21 and produce RF data output signals at the terminal 126.

Also shown in FIG. 1 is a crystal oscillator 22 for providing the principal or fast clock signal for counting operations of digital control and logic circuit 18.

A VCO circuit 24 comprising a voltage controlled oscillator and its associated trimming components which provides the clock frequency for the output pulse widths is also shown in FIG. 1. The VCO circuit 24, upon receiving a VCO ENABLE signal at terminal 130; applies the VCO output signal to the terminal 132 of the digital control and logic circuit 18, the frequency of the VCO output signal being dependent on the then prevailing source voltage Vcc and the digital control and logic circuit 18 produces the VCO ENABLE signal at the terminal 130. The VCO circuit 24 responds and provides the VCO OUT signal at the terminal 132. The digital control and logic circuit 18 responds to the VCO OUT signal to employ the VCO signal as a timing signal in timing the widths of the atrial and ventricular pace initiate signals, thereby effecting an increase in the width of the atrial and ventricular stimulating pulses from the width programmed in memory within the digital control and logic circuit 18. A general operation of the VCO circuit 24 and the digital control and logic circuit 18 for effecting this mode of operation of the device is described in the aforementioned U.S. Patent No. 4,230,120.

The atrial and ventricular output stages 14 and 16 each comprise defibrillation protection diodes 142 and 144 and an output capacitor 134 and 136 respectively connected to the atrial lead terminal 26 and ventricular lead terminal 30. In both instances, the output capacitors are charged to battery voltage B + and are discharged through transistor switches (not shown) within the atrial output stage 150 and the ventricular output stage 152, respectively, and commonly through the conductor 154 and case terminal 28 through the hear tissue and back to the terminals 26 and 30, respectively. The ventricular output capacitor 136 is recharged through conduction of transistor 140 while the stored output capacitor 134 is recharged through the transistors 138, 146, and capacitor 148 and in relation to the voltage on reference capacitor 149. The recharge cycle is under the control of a fast recharge control signal FRV developed by the digital control and logic circuit 18 at terminal 156.

The pulse widths of the atrial and ventricular stimulating pulses are established by the widths of the atrial and ventricular output initiate signals PWA and PWV at terminals 158 and 160 respectively. The atrial and ventricular output stages 150 and 152 are coupled between regulated supply voltage Vcc and system ground through respective biasing components 161 and 162 in a manner well known in the art.

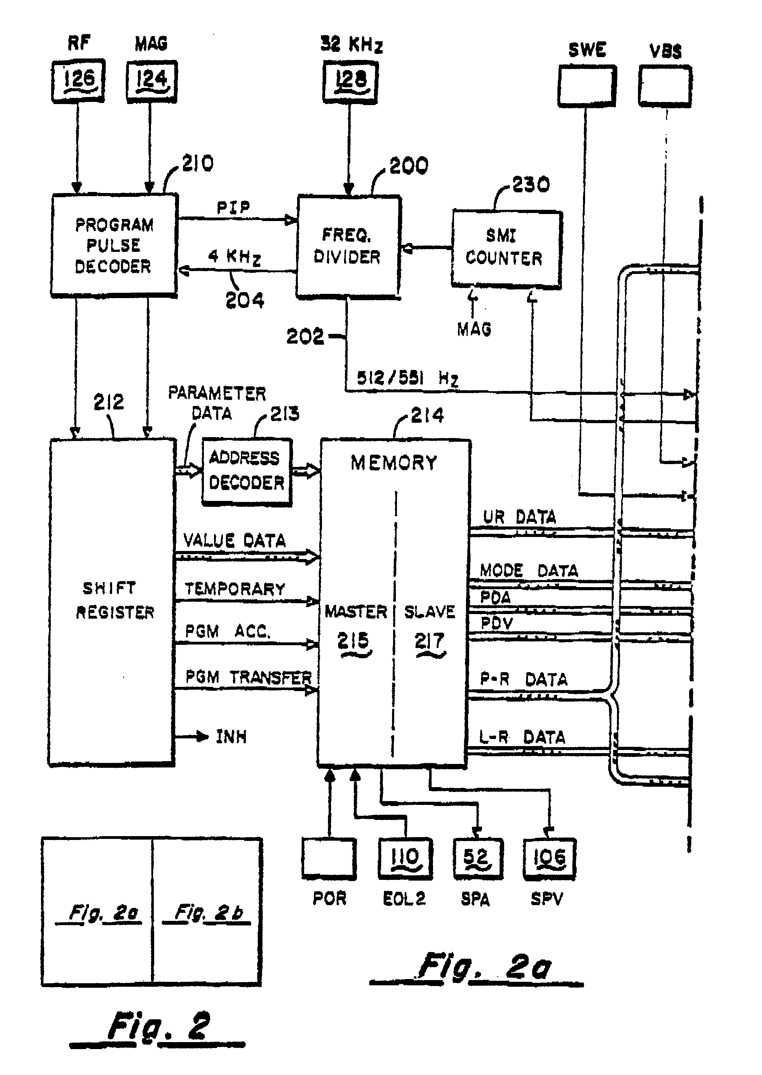

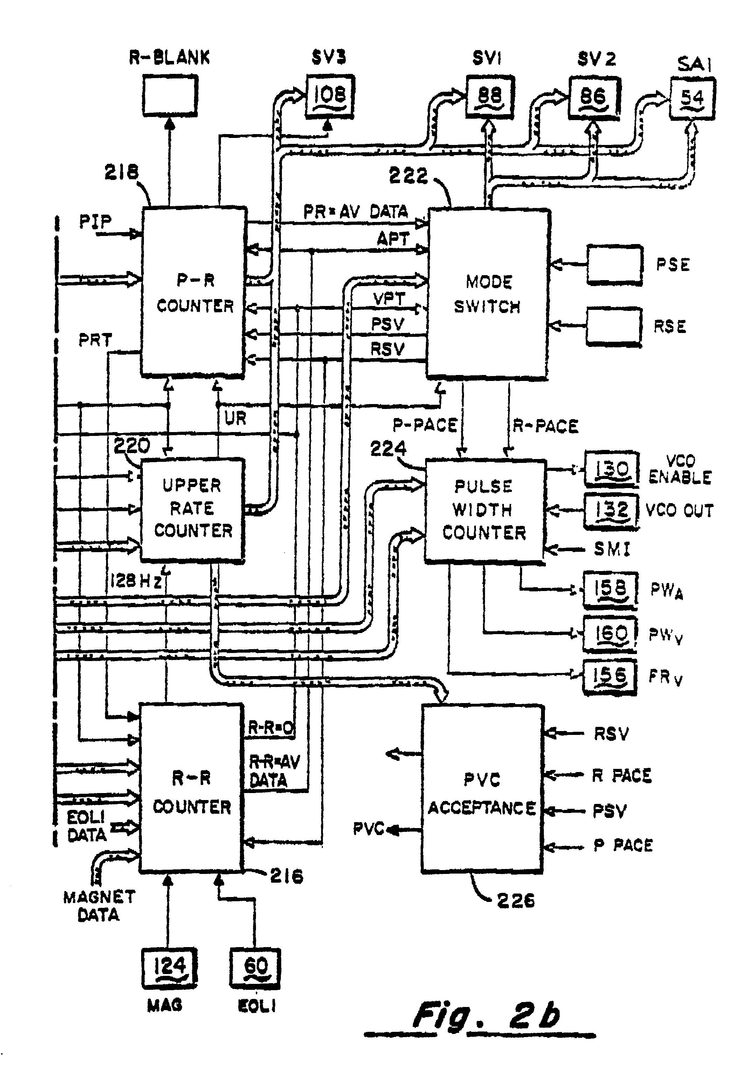

The digital control and logic circuit 18 is depicted in greater detail in FIG. 2. In general terms, FIG. 2 shows the circuit components for receiving externally applied programming signals for programming the modes and parameters of operation of the digitally controlled pacemaker of FIG. 1, memory storage for storing the programming signals, various counting circuits for timing the lower rate escape interval, the upper rate maximum allowed pacing rate, the A-V time delay, and other intervals for setting the pulse width duration of the atrial and ventricular output initiate signals and atrial and ventricular sense amplfiier sensitivities, and switching circuitry for controlling the modes of operation of the pacemaker. The circuit of FIG. 2 is implemented in digital logic components and circuits of said type shown in the aforementioned U.S. Patent No. 4,230,120. Distinctions arise from the fact that the circuit disclosed in that application is intended for control of a ventricular demand or VVI mode pacemaker, and the present invention is implemented in the context of a programmable, fully automatic dual chamber pacemaker normally operable in the DDD mode.

Referring to FIG. 2 in greater detail, the 32 kHz crystal oscillator frequency is applied at terminal 128 to a frequency divider circuit 200 which produces a 512 Hz slow clock signal at terminal 202 and a 4 kHz decoding signal at terminal 204. The 4 kHz signal is applied to the program pulse decoder 210 which decodes incoming RF signals appearing at terminal 126 to separate logic 1 and logic 0 signals from one another. The program pulse decoder 210 applies the decoded data signals to the D input terminal of the shift register 212 and a clocking signal to the C input terminal of the shift register 212. The shift register 212 accumulates the decoded data signals and performs parity checking to assure that the serially received incoming data signals comprise properly formed programming words.

The clock frequency circuit and the content and format of the received data words are similar to that described in the aforementioned Patent No. 4,230,120. In general terms, the decoded data signal at terminal D of shift register 212 is a series of 33 pulse signals separated by short and long intervals defining 32 logic 1 or logic 0 data bits. The 32 bit data words consists of four parts, each of which is 8 bits in length. The four parts are the parameter code, date or value code, access code and parity code and are generated in that order, the least significant bit first. The parameter portion of the data signal defines one of several parameters (including modes of operation) to be modified and whether that modification is to be in a temporary or permanent matter, if that choice is available. The several parameters include "mode" comprising threshold check mode, the fully inhibited mode, the DDD mode, the DVI mode, the VDT/I mode, the VVI mode, the sensitivities of the sense amplifiers, the A-V period, the lower pacing rate, and the upper pacing rate, the atrial and ventricular stimulation pulse widths. Of these parameters, the inhibit and threshold check parameters can only be done in a temporary mode whereas all the others can either be permanently or temporarily programmed. The programming parameter code and value codes are similar to but not necessarily the same as those set forth in the aforementioned U.S. Patent No. 4,230,120, and in any event can be arbitrarily selected by those skilled in the art. The access code will, of course, differ from codes employed to program the pulse generator types. It will be noted that in the context of a dual chamber pacemaker, it will of course be necessary to increase the total number of serial transmissions of the 32 bit word data to effect programming of, for example, the sensitivity of the atrial and ventricular sense amplifiers.

The shift register 212 contains eight access code storing stages, access decoding and parity bit checking logic circuitry for insuring that the access code is correct and that the program word possesses proper parity, five parameter code shift register states for storing the five most significant bits of the eight bit parameter code (the first three bits of the eight bit parameter code are not used whatsoever and are always generated as logic 0 bits), and eight data or value code storage shift register stages for storing eight bits which define a particular value for the parameter selected. Ten further storage register stages and logic circuitry coupled parameters and data storage stages and certain stages of memory 214 effect the transfer of the parameters, e.g., the mode, lower rate, A-V interval, upper rate, atrial pulse width, ventricular pulse width, temporary atrial and ventricular pulse width and atrial and ventricular sense amplifier sensitivities.

A "master" portion of each word is accepted in memory 214 when the access code and parity check are found valid and all 32 bits of the program word is received. Further program transfer logic circuitry within the shift register 212 affects the shifting of the parameter data from the shift register 212 to the "master" register of the memory 214 in the permanent or temporary programming modes described hereinbefore. On acceptance of the transferred word, the shift register stages of shift register 212 are verified to accept the subsequently applied programming signal. Through serial application of the programming signals, all parameters and parameter values are stored within the memory 214, except that (1) if the parameter is "mode" and the value is "inhibit", then the word is not transferred but is applied to the circuit to inhibit all operations; and (2) if the parameter is "sensitivity" and the value code contains the indicator "new value", then only the word can be transferred.

The memory 214 contains address decode logic circuitry, master memory register 215 and slave memory register 217. Each decoded programming word from shift register 212 is transferred into the master register 215. Transfer of all contents of the master register 215 into the slave register 217 occurs only when the last word, indicated by a particular parameter code, has been stored in master portion 215, all the stored words possessed the valid access code and satisfied the parity check, and a ventricular pacing output pulse occurs after the last word and before receipt of any further words.

The master and slave registers also perform the function of temporary and permanent memory in the manner described in the aforementioned U.S. Patent No. 4,230,120 for storing the various parameters and parameter values previously described and effecting the operation of the digital control and logic circuit 18 accordingly. Separate memory stages are devoted to the sensitivity of atrial and ventricular sense amplifiers, the programmable upper rates, the programmable lower rate or basic escape interval, the atrial and ventricular stimulation pulse widths, the A-V delay intervals, and data signifying the mode of operation of the device from among the various modes previously mentioned. The memory 214 furthermore possesses a hard wired lower rate for VVI mode that constitutes a basic safety rate should the power source energy momentarily dip below the circuits minimum operating requirements and also for use as one of the end-of-life test rates. Thus, the pulse generator will operate in a VVI mode at a predetermined rate, for example, 65 beat per minute, and at a predetermined pulse width if either condition exists. The EOL2 signal is applied at terminal 110 to the memory 214 and, under certain circumstances of operation to be described, its presence causes the hard wired safety rate to be substituted for the programmed rate upon magnet mode testing of the battery depletion.

The lower rate data is transferred by bus line from the memory 214 to the R-R or lower rate counter 216. The A-V interval data is transferred by bus to the P-R counter 218 and to the R-R counter 216. The upper rate data is transferred from the memory 214 to the upper rate circuit 220. The pulse width data, PDA and PDV for the control of the atrial and ventricular pulse widths is applied by bus lines from the memory 214 to the pulse width control circuit 224. The upper rate counter 220 is coupled by a bus line to the PVC acceptance circuit 226. The R-R interval counter 216 comprises a ten stage shift register counter at least eight stages of which are coupled through decoding logic circuitry to the memory stages of the memory 214 which specify the programmed lower rate values and the A-V values. The R-R counter stages constitute therefore a presettable down counter, which is downcounted under the control of the 512 Hz slow clock frequency or under the control of a magnet actuated clock frequency of approximately 551 Hz when the safety margin indicator (SMI) counter 230 is activated by the reed switch closure. The SMI counter 230 alters the number of stages of divider 200 to change the slow clock frequency to 551 Hz until it counts three VPT outputs of the R-R counter. The clock frequencies downcount the loaded counter until a first count is reached which, when decoded, provides an atrial pulse trigger signal APT and until a subsequent second count is recorded which, when decoded, provides a ventricular pulse trigger signal VPT. These signals APT and VPT are applied to the P-R counter 218 and the mode switch 222.

Thus during operation of the SMI counter 200, the pacing rate is increased by 10 percent to indicate closure of the reed switch. The SMI operation is therefore similar to that described in greater detail in the aforementioned U.S. Patent No. 4,230,120.

The P-R counter 218 is an up counter that counts the 512 Hz slow clock signal from zero to the by A-V data count in memory 214. Upcounting commences when the signal PSV is received from mode swith 222 or the signal APT is received by the PR counter 218 and ceases when the count reaches a preselected number or the counters are reset by a sensed R wave.

The mode switch 222 also receives the signal APT and VPT and the mode data signals on a bus line from memory 214. The mode signal data sets the logic and switching circuits in the mode switch 222 to the modes previously described. Assuming that the memory 214 is programmed into the DDD mode, for example, the mode switch 222 responds to the signals APT and VPT to produce the P pace and R pace signals that are applied to the pulse duration counter 224. The pulse duration counter 224 responds to the P pace signal and the atrial pulse duration data signal PDA to produce the atrial output initiate signal PWA at terminal 158 and similarly responds to the R pace signal at the ventricular pulse duration data signal DDD to produce the ventricular output initiate signal PWV at the terminal 160.

The output initiate signals PWA and PWV possess the pulse width signified by the programmed pulse duration signals PDA and PDV. In other programmable modes either P pace or the R pace signals are produced according to the mode indicated by the memory mode data. The mode switch 222 also provides signals PSE and RSE that enable or disable the atrial and ventricular sense amplifiers 10 and 12.

Under certain circumstances, the output initiate signals PWA and PWV may possess durations that deviate from the programmed pulse duration signals. This can occur when the battery or power source depletes in energy. The VCO 24 (FIG. 1) automatically changes its frequency with the source voltage, that is, it decreases as the voltage decreases. The VCO output signal at terminal 132 (FIG. 1) is coupled to the PD counter 224 (FIG. 2) and provides its clock signal. Thus, as the frequency slows, the time interval necessary to achieve the PDA or PDV count increases, thereby widening the output initiate pulses PWA and PWV. This operation is again like that described in the aforementioned U.S. Patent No. 4,230,120.

The PVC acceptance circuit 226 responds to premature ventricular contractions sensed within the ventricular escape interval of the R-R counter to provide an output signal to reset the R-R counter timing. The circuit 226 also responds to the atrial and ventricular pacing events (P pace and R pace) developed by the mode switch 222 and the atrial and ventricular sense signals PSV and RSV to provide the ventricular sense amplifier 12 refractory period. The refractory period is decoded by the circuit 226 from the count on the upper rate counter 220, and is ordinarily 233 ms after a paced or sensed ventricular event. But, if a PVC is sensed and triggers circuit 226, then the upper rate counter count is decoded to provide a 342 ms refractory period.

In addition, if a P wave is sensed (PSV signal) following two successive sensed R waves (two RSV signals) or ventricular output pulses (R pace signals) or following an R pace and RSV signal, then the circuit 226 further establishes a 342 ms A-V delay interval commenced on the subsequently sensed atrial depolarization. This prolongation is employed to prevent a ventricular output pulse triggered by a sensed P-wave from possibly falling within the heart's vulnerable zone following the preceding premature ventricular contraction. This operation is further described in my commonly assigned copending U.S. Patent Application Serial N° 235,232, filed 17 February, 1981.

Whenever the R-refractory is retriggered for 342 ms, the A-V counter is also disabled for 342 ms (bigeminy A-V disable). In case the device is programmed to VVI or DVI mode, ventricular amplifier outputs after ventricular blanking and before refractory ends cause another 125 ms ventricular blanking timer and the ventricular refractory period to be set again to 233 ms. Thus the digital reversion window will be equal to 108 ms.

This pacemaker as described hereinbefore has sense amplifiers 10, 12 and pulse output circuits 14, 16 for connection to both the ventricle and atrium. Two unipolar electrodes are coupled to terminals 26 and 30 and the can 28 of the pulse generator serves as a common indifferent electrode. The device can be progammed to operate in the DDD, DVI, VDT/I and VVI mode, but may have numerous resultant modes depending on the programming of the output pulse widths and amplifier sensitivities and on the natural underlying heart rhythm. Such resultant modes can include DAT/I, DVI, DOO, AOO, VOO, VAT, AVI, AAI, ADT/I and OOO.

In the DDD mode, the programmable lower rate establishes the rate at which the pulse generator will pace if no P or R waves are sensed. At this rate the operation is A-V sequential and the interval between atrial and ventricular outputs (the A-V interval) is programmable.

If a P-wave is sensed, it will inhibit an atrial pacing output and trigger the A-V interval circuitry. If an R-wave is sensed prior to the completion or the A-V interval, the ventricular pacing output is inhibited and all timing circuits will be reset. If an R-wave is not sensed prior to the completion of the A-V interval, a ventricular pacing output will occur at the end of the delay.

After an atrial event (sensed P-wave or atrial pacing output) the atrial amplifier is blanked. The ventricular amplifier 12 is also blanked for 15, 6 ms (ventricular blanking) after an atrial output to avoid sensing the voltage induced on the ventricular lead. The atrial amplifier 10 remains off until after the next ventricular event and is turned on only after the ventricular amplifier 12 has been on for 31 ms. This is to help insure that when both amplifiers are being turned back on that possible ectopic events which may occur are sensed first by the ventricular amplifier or are not sensed at all. If both amplifiers are turned on simultaneously, there exists the possibility that a heat depolarization could have just passed by the ventricular electrode and will not be sensed by that amplifier. However, that same event could reach the atrial electrodes later and be regarded as a P-wave starting the A-V interval and causing a ventricular stimulating pulse. Such ectopic events that occur high in the ventricle are likely to be sensed in the atrium. The consequence of the PVC sensed as a P-wave could be to pace one A-V interval later into what may be the repolarization phase of the PVC if the PVC is not sensed by the ventricular sense amplifier. The atrial blanking overlap is described in greater detail in commonly assigned, co-pending U.S. Patent Application Serial No. 145,052, filed April 30, 1980, by Harold Toby Markowitz.

A P-wave sensed after the atrial amplifier 10 is turned on out before the end of the upper rate period will trigger an A-V interval by the P-R counter 218; however, the pulse gnerator will wait until the end of the upper rate period before a ventricular output will occur. This implements the upper rate characteristic of the atrial triggering mechanism.

At the time of a ventricular event (sensed R-wave or ventricular pacing output) the ventricular amplifier 12 is turned off or blanked for 125 ms, the upper rate period is started by the upper rate timer/counter 220 (at rates of 100, 125, 150, or 175 bpm) and the 233 ms ventricular refractory is started. During refractory the lower rate timer 216 cannot be reset. The timer 216 will be reset by either a ventricular pacing output or sensed R-wave which occurs outside of the ventricular refractory period. After the ventricular amplifier 12 is turned on and before the end of the 233 ms R-refractory period, a ventricular sense amplifier output will restart the 233 ms refractory period, 125 ms R-blanking, 156 ms atrial blanking and upper rate period.

In the programmed modes DDD or VDT/I, the ventricular amplifier outputs after ventricular blanking and before refractory ends cause another 125 ms ventricular blanking, 156 ms atrial blanking and the ventricular refractory timer to be set to 342 ms (PVC refractory) provided that no preceeding P-wave has been sensed. Thus, there is a window of $342-125 = 217$ ms for a retriggering of refractory. In order for a signal of continuous interference to keep retriggering the venticular refractory timer (digital reversion) the ventricular amplifier outputs must be no further apart than 342 ms. If the ventricular refractory timer is always retriggered during the 217 ms window then the pacemaker will operate A-V sequential asynchronous (DOO) because the lower rate timer will not be reset from ventricular amplifier outputs. This corresponds to a lowest interference frequency of 2.9 Hz.

However, if the ventricular amplifier output, after ventricular blanking and before refractory end, was preceeded by a sensed P-wave, the R-refractory timer will be set to 233 ms as described hereinbefore.

The pacemaker programmed in the DDD mode will perform in a pseudo-Wenckebach fashion when atrial rates exceed the programmed upper rate. There will be gradual A-V lengthening until an atrial beat does not lead to a ventricular pacing output. The process then repeats. At high upper rates the device will reach 2:1 block dependent upon the programmed A-V interval.

Thus, at a setting of 250 ms A-V interval and 175 bpm upper rate, the device will operate in a 2:1 block at an atrial rate of 148 bpm (ventricular rate = 74 bpm) and the upper rate will not be used. If the upper rate were set to 125 bpm the device would exhibit the A-V prolongation/dropped beat characteristic from atrial rates of 125 to 148 bpm. At 148 bpm the ventricular rate would drop to half that of the atrial input. Shorter A-V settings push the 2:1 block point to higher rates. This operation is similar in principle to that shown in commonly assigned U.S. Patent No. 4,059,116.

This description is presented to describe the best mode in which the following description of the invention may be put into practice.

## Claims

1. A programable medical device including:

(a) an atrial output stage (14) for providing atrial stimulating pulses at a recurring rate interval, said output stage (14) including an atrial capacitor (134);

(b) means for detecting ventricular depolarizations (12);

(c) means for applying external programming signals to the programmable device for programming variable operational parameters and variable modes (32, 124, 126);

(d) programmable digital control means (18) including

(i) means for storing (Figure 2 - 212, 214) variable digital data derived from said externally applied programming signals corresponding to a lower rate at which the pulse generator will pace if neither P (atrial) waves nor R (ventricular) waves are sensed within the escape intervals established by said lower rate data and for storing the desired programmable interval between the production of an atrial and a subsequent ventricular stimulation pulse (A-V interval);

(ii) means for providing (Figure 2 - 216, 218, 222, 224, APT, P-pace) an atrial pace initiate signal (PWA) to said atrial output stage (14) to initiate an atrial stimulating pulse if no naturally occurring P wave is sensed within the escape interval established by the lower rate data, the atrial stimulating pulse being the discharge of said atrial capacitor (134);

(iii) means for providing and applying a control signal (SVI, SV2, Figure 2b - 218, 220, 222, 86, 88) to the means for detecting ventricular depolarizations (12) to disable this detecting means for a predetermined period of time starting in timed relation to the generation of an atrial pace initiate pulse (PWA), so that the means for detecting ventricular depolarizations (12) cannot sense said atrial stimulation pulse and mistake it for a ventricular depolarization, and

(iv) means (Figure 2b - 224) for generating a fast recharge control signal (Figures 1b and 2b - FRV, 156) to control the recharge cycle.

## Revendications

1. Dispositif médical programmable comprenant:

(a) un étage de sortie atrial (14) servant à délivrer des impulsions de stimulation atriale à un intervalle de récurrence, ledit étage de sortie (14) comprenant un condensateur atrial (134);

(b) des moyens pour détecter des dépolarisations ventriculaires (12);

(c) des moyens pour appliquer des signaux externes de programmation au dispositif programmable pour la programmation de paramètres de fonctionnement variables et de modes variables (32, 124, 126);

(d) les moyens de commande numérique programmables (18) comprenant

(i) des moyens pour mémoriser (figure 2 - 212, 214) des données numériques variables obtenues à partir desdits signaux de programmation appliqués de l'extérieur et correspondant à une cadence réduite, avec laquelle le générateur d'impulsions applique sa stimulation si aucune onde P (atriale) ni aucune onde R (ventriculaire) ne sont détectées pendant les intervalles de libération établis par lesdites données à cadence réduite, et pour mémoriser l'intervalle programmable désiré entre la production d'une impulsion de stimulation atriale et d'une impulsion ultérieure de stimulation ventriculaire (intervalle A-V);

(ii) des moyens pour envoyer (figure 2 - 216, 218, 222, 224, APT, stimulation P) un signal (PW) de déclenchement de la stimulation atriale audit étage de sortie atrial (14) pour déclencher une impulsion de stimulation atriale si aucune onde P, qui apparaît naturellement, n'est détectée pendant l'intervalle de libération établi par les données à cadence réduite, l'impulsion de stimulation atriale étant la décharge dudit condensateur atrial (134);

(iii) des moyens pour produire et appliquer le signal de commande (SVI, SV2, figure 2b - 218, 220, 222, 86, 88) aux moyens servant à détecter des dépolarisations ventriculaires (12) pour invalider ces moyens de détection pendant un intervalle de temps prédéterminé qui commence, d'une manière associée dans le temps, à la production d'une impulsion (PWA) de déclenchement de la stimulation atriale, de sorte que les moyens servant à détecter les dépolarisations ventriculaires (12) ne permettent pas de détecter ladite impulsion de stimulation atriale et la confondent avec une dépolarisation ventriculaire, et

(iv) des moyens (figure 2b - 224) pour détecter un signal de commande de recherche rapide (figures 1b et 2b -FRV, 156) pour commander le cycle de recharge.

## Patentansprüche

1. Programmierbares medizinisches Gerät mit:

(a) einer atrialen Ausgangsstufe (14) zum Anliefern von atrialen Reizimpulsen zu einem wiederkehrenden Ratenintervall, wobei die Ausgangsstufe (14) einen atrialen Kondensator (134) aufweist;

(b) einer Anordnung zum Erkennen von ventrikulären Depolarisationen (12);

(c) einer Anordnung zum Anlegen von externen Programmiersignalen an das programmierbare Gerät zum Programmieren von variablen Betriebsparametern und variablen Betriebsarten (32,124,126); und

(d) einer programmierbaren digitalen Steueranordnung (18), die versehen ist mit

(i) einer Anordnung zum Speichern (Fig. 2 - 212, 214) von variablen digitalen Daten, die von extern angelegten Programmiersignalen abgeleitet sind, welche einer niedrigeren Rate entsprechen, mit welcher der Impulsgenerator Schrittmacherimpulse abgibt, wenn weder P-(Vorhof-)Wellen noch R-(Kammer-)Wellen innerhalb der von den niedrigeren Ratendaten bestimmten Verlaufsintervalle erfaßt werden, und zum Speichern des erwünschten programmierbaren Intervalls zwischen dem Erzeugen eines atrialen und eines nachfolgenden ventrikulären Reizimpulses (AV-Intervall);

(ii) einer Anordnung zum Anliefern (Fig. 2 - 216, 218, 222, 224, APT, P-pace) eines atrialen Schrittmacherauslösesignals (PWA) an die atriale Ausgangsstufe (14), um einen atrialen Reizimpuls einzuleiten, wenn keine natürlich auftretende P-Welle innerhalb des von den niedrigeren Ratendaten bestimmten Verlaufsintervalls erfaßt wird, wobei der atriale Reizimpuls in der Entladung des atrialen Kondensators (134) besteht;

(iii) einer Anordnung zum Anliefern und Anlegen eines Steuersignals (SV1 SV2, Fig. 2b - 218, 220, 222, 86, 88) an die Anordnung zum Erkennen von ventrikulären Depolarisationen (12), um diese Erkennungsanordnung für eine vorbestimmte Zeitspanne zu sperren, die in zeitlicher Beziehung zu dem Erzeugen eines atrialen Schrittmacherauslöseimpulses (PWA) beginnt, so daß die Anordnung zum Erkennen von ventrikulären Depolarisationen (12) den atrialen Reizimpuls nicht erfaßt und fälschlich als eine ventrikuläre Depolarisation werten kann; und

(iv) einer Anordnung (Fig. 2b - 224) zum Erzeugen eines Raschwiederauflade-Steuersignals (Fign. 1b und 2b - FRV, 156) zum Steuern des Wiederaufladezyklus.

ATRIAL SENSE AMPLIFIER

VENTRICULAR SENSE AMPLIFIER

Fig. 1

Fig. 1b

Fig. 1a

Fig. 1

Fig. 1a

Fig. 1b

EP 0 160 801 B1

Fig. 2a

Fig. 2

*Fig. 2b*